(19) Europäisches Patentamt / European Patent Office / Office européen des brevets

(11) **EP 3 205 211 B1**

(12) **EUROPEAN PATENT SPECIFICATION**

(45) Date of publication and mention
of the grant of the patent:
**02.11.2022 Bulletin 2022/44**

(21) Application number: **17160863.1**

(22) Date of filing: **28.08.2012**

(51) International Patent Classification (IPC):
*A01P 1/00* (2006.01)    *A01N 31/02* (2006.01)
*A01N 37/36* (2006.01)    *A01N 43/16* (2006.01)
*A61K 31/045* (2006.01)    *A61K 31/047* (2006.01)
*A61K 31/164* (2006.01)    *A61K 31/355* (2006.01)
*A61K 8/34* (2006.01)    *A61K 8/67* (2006.01)
*A61K 8/42* (2006.01)    *A61Q 17/00* (2006.01)
*A61K 31/197* (2006.01)

(52) Cooperative Patent Classification (CPC):
(C-Sets available)
**A01N 31/02; A61K 8/34; A61K 8/345; A61K 8/42;
A61K 8/678; A61K 31/045; A61K 31/197;
A61K 31/355; A61Q 17/005;** Y02A 50/30    (Cont.)

(54) **ALCOHOL-BASED ANTIVIRAL COMPOSITION**

AUF ALKOHOL BASIERENDE ANTIVIRALE ZUSAMMENSETZUNG

COMPOSITION ANTIVIRALE À BASE D'ALCOOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**16.08.2017 Bulletin 2017/33**

(62) Document number(s) of the earlier application(s) in
accordance with Art. 76 EPC:
**12753706.6 / 2 890 242**

(73) Proprietor: **Ecolab USA Inc.
St. Paul, MN 55102 (US)**

(72) Inventors:
• **Teusch, Katja
41539 Dormagen (DE)**
• **Jäger, Stefan
50733 Köln (DE)**
• **Meyer, Bernhard
40822 Mettmann (DE)**
• **Denzin, Silke
40764 Langenfeld (DE)**

(74) Representative: **Michalski Hüttermann & Partner
Patentanwälte mbB
Kaistraße 16A
40221 Düsseldorf (DE)**

(56) References cited:
EP-A1- 0 176 720        WO-A1-99/61045
US-A- 5 114 957        US-A1- 2006 074 029
US-A1- 2008 293 825        US-A1- 2009 226 498

• **SNIPES W ET AL: "INACTIVATION OF
LIPID-CONTAINING VIRUSES BY LONG-CHAIN
ALCOHOLS", ANTIMICROBIAL AGENTS AND
CHEMOTHERAPY, AMERICAN SOCIETY FOR
MICROBIOLOGY, US, vol. 11, no. 1, 1 January
1977 (1977-01-01), pages 98-104, XP009034119,
ISSN: 0066-4804**

EP 3 205 211 B1

(52) Cooperative Patent Classification (CPC): (Cont.)

C-Sets
**A01N 31/02, A01N 37/36, A01N 43/16;**
**A61K 31/045, A61K 2300/00;**
**A61K 31/197, A61K 2300/00;**
**A61K 31/355, A61K 2300/00**

**Description**

Field of the Invention

The invention relates to a virucidal composition, especially for the virucidal disinfection of hands, and to a method for the virucidal disinfection of mammalian skin.

Background of the Invention

Skin disinfectants especially for disinfecting hands are used in several fields where a contamination with viruses should be avoided. Many viruses, e.g. influenza, have viral envelopes covering their protein capsids. These viruses can normally be easily inactivated by compounds destroying this lipoid envelope. In contrast, non enveloped viruses, which do not have a lipoid envelope, such as poliovirus are quite more stable against conventional disinfectants and constitute an important class of medically significant pathogens. There are three types of poliovirus and many strains of each type. Type 1 poliovirus is the most virulent and common.

For the classification of virucidal disinfectants, in the European standard EN14476 (February 2007) the test methods, the test viruses, and the conditions for measuring the inactivation of specific test viruses are described. According to this standard hand disinfectants are classified as virucidal if they can inactivate certain viruses within a certain time and to a specific extent. According to the European standard EN 14476 poliovirus and adenovirus can be used as test virus for virucidal compositions for hand disinfection. The standard EN 14476 also describes the test procedure and the way of measuring the inactivation of the test viruses. If a virucidal disinfectant inactivates the test virus according to the standard EN14476 within a given contact time by 4 $\log_{10}$ units, the inactivation of the virus is 99.99 %.

A non-enveloped virus which is very difficult to inactivate is poliovirus. In virucidal disinfectants usually high concentrations of ethanol are necessary to inactivate these non enveloped viruses such as poliovirus. A disadvantage is that ethanol evaporates very fast and that the necessary ethanol concentration decreases during the treatment to a level which is no longer high enough to inactivate the viruses sufficiently. For example, after one minute contact time a high ethanol concentration can decrease to between one forth and one third lower than at the beginning of the treatment. But normally contact times of at least two minutes are required for sufficient virucidal effect. As a consequence, it is necessary to apply an ethanol based virucidal composition again after a contact time of one minute to avoid a decrease in the ethanol concentration because of evaporation from the skin.

Document WO 2011/072728 describes an ethanol-based virucidal composition in form of a gel to reduce the evaporation during use of the virucidal composition on the skin.

Still, numerous liquid compositions are on the market. A product often used is for example Sterillium Virugard® (Bode Chemie, Hamburg). This product is based on 95% ethanol and further contains butanone, glycerol, myristyl alcohol and petrol ether. The corresponding document is EP 0 176 720 A1 which describes a disinfectant for naked viruses comprising at least 70% methanol and/or ethanol and 1 to 10% glycerol. However, the product Sterillium Virugard® contains a high concentration of 95% ethanol which suffers rapid evaporation. Further disadvantageously, if the product is used frequently the skin desiccates and starts itching.

US 5114957 A relates to methods for inhibiting viral and retroviral replication and for treating viral and retroviral infections via the administration of compositions containing Vitamin E, tocopherol, or a tocopherol derivative and, more particularly, compositions containing α-tocopherol or a pharmaceutically effective prodrug thereof.

WO 99/61045 relates to a topical drug for the treatment of viral infections, containing a first active ingredient which inhibits the multiplication of viruses in a host cell. The drug in addition contains at least one second active ingredient which inhibits the penetration of the viruses into other host cells. The document also relates to uses for such a drug.

US 2008/0293825 A1 pertains to an alcohol based antimicrobial skincare composition. The document also pertains to an alcohol based antimicrobial skincare composition with skin health benefits including moisturization and skin barrier maintenance effects. The document further pertains to an alcohol based antimicrobial skincare composition that is a stable emulsion that has a certain viscosity to it that provides containment, for example on a user's hands. Finally, the document pertains to an alcohol based antimicrobial skincare composition that may be used in the healthcare industry, for example as a surgical scrub, healthcare personnel handwash, or patient pre-operative site preparation.

US 2009/0226498 A1 generally relates to moisturizing hand sanitizers including alcohols that are effective in killing microorganisms while providing a moisturizing benefit to the user's skin. More particularly, the alcohol-based hand sanitizers include a high internal phase emulsion which allows moisturizers or skin protectants such as emollients and/or silicones to be stably incorporated into the sanitizer.

US 2006/0074029 A1 describes an extended resident topical antimicrobial composition comprising an active antimicrobial and moisturizing ingredients that meets antimicrobial efficacy requirements and improves the condition of the user's skin as well. The composition achieves desired antimicrobial efficacy and therapeutic effect and still exhibits a relatively short dry time to avoid unpleasant feel. The composition comprises: a) an antimicrobial agent; and b) a moisturization component comprising a combination of a saccharide isomerate and carbohydrate complex together with glycerin. In a preferred embodiment, the antimicrobial agent is a broad spectrum antimicrobial agent such as alcohol. The composition is particularly useful to healthcare workers, whose usage of hand sanitizers can be on a daily and frequent basis.

Therefore, the object underlying the present invention was to provide a virucidal disinfectant having sufficient activity against poliovirus, as well as having a low ethanol concentration.

Summary of the Invention

It is provided a virucidal composition comprising:

- equal or more than 70 wt.-% to less than 95 wt.-% of a C2 to C3 monoalcohol;
- equal or more than 0.01 wt.-% to equal or less than 0.5 wt.-% of tocopheryl or tocopheryl acetate;
- equal or more than 0.01 wt.-% to equal or less than 1 wt.-% of panthenol;
- equal or more than 0.1 wt.-% to equal or less than 5 wt.-% of glycerol;
- equal or more than 0.01 wt.-% to equal or less than 5 wt.-% of myristyl alcohol.

It has been surprisingly found that the efficacy of the virucidal composition according to the invention against poliovirus is increased by the addition of the excipients although comprising a rather low amount of ethanol. The present invention has the advantage of providing a fast acting hand disinfectant with excellent skin compatibility. The virucidal composition is suitable for skin and hand disinfection and inactivates a most resistant virus but also is skin-compatible even if frequently used. Hence, the virucidal composition fulfils two conditions which are quite difficult to meet.

In accordance with another aspect of the present invention is provided the virucidal composition for use in a method for the virucidal disinfection of mammalian skin comprising the steps of:

- providing the virucidal composition of the invention, and
- contacting the skin with the virucidal composition for at most 2 min contact time, preferably for at most 1 min contact time, wherein the virus titre is reduced for at least 4 $\log_{10}$ reduction steps measured according to EN 14476.

Definitions

The term "virucidal disinfection" as used herein means an inactivation of the test virus by at least 4 log units in at most two minutes contact time under the conditions and methods according to the European standard EN14476 (February 2007).

Weight percent, weight-% or wt.-% are synonyms that refer to the concentration of a substance as the weight of the substance divided by the weight of the composition and multiplied by 100. The weight-% (wt.-%) of the components are calculated based on the total weight amount of the composition, if not otherwise stated.

The total amount of all components of the composition does not exceed 100 wt.-%. The remainder up to 100 wt.-% of the virucidal composition of the invention can be water. The water content of the virucidal composition is simply determined by subtracting the amounts of all the other ingredients from 100 wt.-%.

All numeric values are herein assumed to be modified by the term "about", whether or not explicitly indicated. As used herein, the term "about" refers to variation in the numerical quantity that can occur, for example, through typical measuring and liquid handling procedures used for making concentrates or use solutions in the real world; through inadvertent error in these procedures; through differences in the manufacture, source, or purity of the ingredients used to make the compositions or carry out the methods; and the like. The term "about" also encompasses amounts that differ due to different equilibrium conditions for a composition resulting from a particular initial mixture. Whether or not modified by the term "about", the claims include equivalents to the quantities.

It should be noted that, as used in this specification and the appended claims, the singular forms "a", "an" and "the" include plural referents unless the content clearly dictates otherwise.

Thus, for example, reference to a composition containing "a compound" includes a mixture of two or more compounds.

Detailed Description of the Invention

Surprisingly it has been discovered that the virucidal composition comprising:

- equal or more than 70 wt.-% to less than 95 wt.-% of a C2 to C3 monoalcohol;
- equal or more than 0.01 wt.-% to equal or less than 0.5 wt.-% of tocopheryl or tocopheryl acetate;

- equal or more than 0.01 wt.-% to equal or less than 1 wt.-% of panthenol;
- equal or more than 0.1 wt.-% to equal or less than 5 wt.-% of glycerol;
- equal or more than 0.01 wt.-% to equal or less than 5 wt.-% of myristyl alcohol, provides inactivation activity against poliovirus faster than pure ethanol although comprising a rather low amount of alcohol, particularly ethanol. Virucidal compositions which have an acceptable inactivation activity against poliovirus known from the state of the art usually contain far higher amounts of alcohol.

According to one embodiment, the virucidal composition comprises from equal or more than 0.5 wt.-% to equal or less than 1.5 wt.-%, further preferred from equal or more than 0.8 wt.-% to equal or less than 1 wt.-%, myristyl alcohol.

1-Tetradecanol commonly is denoted myristyl alcohol. It was found that a virucidal composition comprising ethanol, tocopheryl acetate, panthenol, glycerol, and myristyl alcohol in comparison to pure ethanol of the same concentration achieved superior virucidal efficacy against poliovirus. Especially, the virucidal composition provides sufficient inactivation of poliovirus by at least 4 $\log_{10}$ units within one minute contact time. This provides a very fast acting virucidal composition which for example can advantageously be used as a hand disinfectant.

The virucidal composition can comprise skin care components. According to another embodiment, the virucidal composition comprises:

- equal or more than 0.01 wt.-% to equal or less than 1 wt.-% of isopropyl palmitate;
- equal or more than 0.01 wt.-% to equal or less than 1 wt.-% of aloe vera;
- equal or more than 0.01 wt.-% to equal or less than 1 wt.-% of C12-15 alkyl benzoate;
- equal or more than 0.01 wt.-% to equal or less than 0.5 wt.-% of polydimethylsiloxane.

The term "C12-15 alkyl benzoate" as used herein means the ester of benzoic acid and C12-15 alcohols. The C12-15 alcohols can be a mixture of C12 to C15 primary and branched alcohols. Polydimethylsiloxane (PDMS) is a polymeric organosilicon according to the formula $CH_3[Si(CH_3)_2O]_nSi(CH_3)_3$ wherein n is the number of repeating monomer $[SiO(CH_3)_2]$ units, and is occasionally denoted dimethicone.

It was found that a virucidal composition comprising tocopheryl acetate, panthenol, glycerol, isopropyl palmitate, aloe vera, alkyl benzoate and polydimethylsiloxane but only 85 wt.-% of ethanol achieved comparable virucidal efficacy as pure ethanol at a higher concentration. This provides a virucidal composition with excellent skin compatibility, which advantageously can be used as a hand disinfectant for frequent use.

The virucidal composition according to the invention has a broad virucidal activity and is particularly skin-compatible. In addition, the virucidal composition meets the obligations of the European standard EN 14476 (2007) and achieves an inactivation of the test viruses poliovirus and adenovirus by at least 4 $\log_{10}$ units in at most two minutes contact time according to the test procedure described in EN 14476 (2007).

The virucidal composition can inactivate the virus titre of poliovirus (type 1 strain LSc-2ab) within 2 min contact time by at least 4 $\log_{10}$ reduction steps. Preferably, the virucidal composition can inactivate the virus titre of poliovirus (type 1 strain LSc-2ab) within 1 min contact time by at least 4 $\log_{10}$ reduction steps.

The C2 to C3 monoalcohol can be selected from the group consisting of ethanol, 1-propanol and 2-propanol or mixtures thereof. In a preferred embodiment the monoalcohol is ethanol. According to one embodiment, the virucidal composition comprises from equal or more than 70 wt.-% to less than 95 wt.-% of ethanol. According to another embodiment, the virucidal composition comprises from equal or more than 80 wt.-% to less than 95 wt.-% of ethanol. According to another embodiment, the virucidal composition comprises from equal or more than 80 wt.-% to equal or less than 92 wt.-% of ethanol. According to another embodiment, the virucidal composition comprises from equal or more than 85 wt.-% to equal or less than 90 wt.-% of ethanol. In embodiments, the virucidal composition can comprise 84 wt.-%, 85 wt.-% or 89 wt.-% of ethanol.

The tocopheryl derivative can be tocopheryl acetate. The virucidal composition can comprise from equal or more than 0.02 wt.-% to equal or less than 0.1 wt.-%, preferably from equal or more than 0.04 wt.-% to equal or less than 0.06 wt.-%, of tocopheryl acetate. The virucidal composition can comprise from equal or more than 0.1 wt.-% to equal or less than 0.2 wt.-%, preferably from equal or more than 0.13 wt.-% to equal or less than 0.16 wt.-%, of panthenol. The virucidal composition can comprise from equal or more than 0.2 wt.-% to equal or less than 4 wt.-%, preferably from equal or more than 0.4 wt.-% to equal or less than 2 wt.-%, more preferably from equal or more than 0.4 wt.-% to equal or less than 0.6 wt.-%, of glycerol.

According to one embodiment, the virucidal composition comprises:

- equal or more than 80 wt.-% to equal or less than 92 wt.-% of ethanol;
- equal or more than 0.01 wt.-% to equal or less than 0.5 wt.-% of tocopheryl acetate;
- equal or more than 0.01 wt.-% to equal or less than 1 wt.-% of panthenol;
- equal or more than 0.1 wt.-% to equal or less than 5 wt.-% of glycerol;

EP 3 205 211 B1

- equal or more than 0.5 wt.-% to equal or less than 1.5 wt.-% of myristyl alcohol.

According to a further embodiment, the virucidal composition comprises:

- equal or more than 85 wt.-% to equal or less than 90 wt.-% of ethanol;
- equal or more than 0.04 wt.-% to equal or less than 0.06 wt.-% of tocopheryl acetate;
- equal or more than 0.13 wt.-% to equal or less than 0.16 wt.-% of panthenol;
- equal or more than 0.4 wt.-% to equal or less than 2 wt.-%, preferably from equal or more than 0.4 wt.-% to equal or less than 0.6 wt.-%, of glycerol;
- equal or more than 0.8 wt.-% to equal or less than 1 wt.-% of myristyl alcohol.

A virucidal composition comprising ethanol, tocopheryl acetate, panthenol, glycerol, and myristyl alcohol in comparison to pure ethanol of the same concentration provides superior virucidal efficacy against poliovirus, especially an inactivation of poliovirus by at least 4 $\log_{10}$ units within one minute contact time.

According to one embodiment, the virucidal composition comprises:

- equal or more than 0.2 wt.-% to equal or less than 0.5 wt.-% of isopropyl palmitate;
- equal or more than 0.2 wt.-% to equal or less than 0.8 wt.-% of aloe vera;
- equal or more than 0.1 wt.-% to equal or less than 0.5 wt.-% of C12-15 alkyl benzoate;
- equal or more than 0.01 wt.-% to equal or less than 0.1 wt.-% of polydimethylsiloxane.

According to a further embodiment, the virucidal composition comprises:

- equal or more than 0.3 wt.-% to equal or less than 0.4 wt.-%, of isopropyl palmitate;
- equal or more than 0.4 wt.-% to equal or less than 0.6 wt.-%, of aloe vera;
- equal or more than 0.2 wt.-% to equal or less than 0.3 wt.-%, of C12-15 alkyl benzoate;
- equal or more than 0.01 wt.-% to equal or less than 0.08 wt.-%, of polydimethylsiloxane.

A virucidal composition comprising tocopheryl acetate, panthenol, glycerol, isopropyl palmitate, aloe vera, alkyl benzoate and polydimethylsiloxane but only 85 wt.-% of ethanol can achieve comparable virucidal efficacy as pure ethanol at higher concentration. Further, a virucidal composition comprising tocopheryl acetate, panthenol, glycerol, isopropyl palmitate, aloe vera, alkyl benzoate and polydimethylsiloxane provides excellent skin compatibility.

The virucidal composition further can comprise lactic acid. According to a further embodiment, the virucidal composition comprises from equal or more than 0.001 wt.-% to equal or less than 0.5 wt.-%, preferably from equal or more than 0.001 wt.-% to equal or less than 0.1 wt.-%, further preferred from equal or more than 0.002 wt.-% to equal or less than 0.01 wt.-%, lactic acid. The virucidal composition only comprises low amounts of lactic acid. A low amount of lactic acid provides for a low acidity of the composition which allows for a skin-compatible pH value of the virucidal composition. In contrast, high amounts of acids which often are used as a virucidal enhancer in compositions of the state of the art damage the skin especially if the composition is more frequently used. Hence, an additional advantage of the virucidal composition according to the invention is that the pH value is skin friendly, and as a consequence the skin is not irritated even if the composition is used frequently.

The virucidal composition according to the invention preferably does not contain percarboxylic acid. Percarboxylic acids damage the skin.

The virucidal composition according to the invention is prepared by usual production processes, for example by mixing together the components of the virucidal composition in a mixing device. The virucidal composition is normally in the form of a liquid. However, for specific uses the virucidal composition can also be in the form of a gel, a foam, or an emulsion. The composition can further comprise additives selected from the group consisting of stabilisers, fragrance, colorants, emulsifiers, thickeners, neutralizers, denaturants, wetting agents, or mixtures thereof. The virucidal composition can comprise from equal or more than 0.01 wt.-% to equal or less than 0.2 wt.-% fragrance, for example 0.07 wt.-% fragrance. It should be understood that additives are optional components and can be omitted.

The virucidal composition especially is usable for the inactivation of poliovirus (type 1 strain LSc-2ab). The virucidal composition according to the invention is highly active against poliovirus. Especially a virucidal composition comprising ethanol, tocopheryl acetate, panthenol, glycerol, and myristyl alcohol is capable of reducing the virus titre of Poliovirus type 1 by at least 4 $\log_{10}$ reduction steps within a minute. This is particularly advantageous as usual compositions of the state of the art for the inactivation of poliovirus need longer contact periods to achieve the same 4 $\log_{10}$ reduction.

The virucidal composition can be used for the virucidal disinfection of human or animal skin. Especially, the virucidal composition is usable for the virucidal disinfection of hands. The virucidal composition fulfils the European testing standard EN14476. Hand disinfection is important for example in surgery or nursery. Hand disinfectants are normally used in hospitals, nursing homes, and in the surgical field. Particularly in the medical field it is especially advantageous that even after a short contact period the virus titre is sufficiently reduced. Furthermore the virucidal composition is highly compatible to skin even if used very frequently during a day as is essential for example in hospitals. The composition can be used as virucidal hand disinfectant for frequent use. The virucidal composition also can be used in the field of food processing, for example meat and/or poultry processing, or in the processing of beverages.

The virucidal composition is suitable for the virucidal disinfection of various virus-contaminated surfaces. The virus-contaminated surface preferably is human or animal skin. The invention further relates to the virucidal composition for use in a method for the virucidal disinfection of mammalian skin comprising the steps of:

- providing the virucidal composition according to the invention, and
- contacting the skin with the virucidal composition for at most 2 min contact time wherein the virus titre is reduced for at least 4 $\log_{10}$ reduction steps measured according to EN 14476.

In an embodiment, the virus is poliovirus (type 1 strain LSc-2ab). In an embodiment, the skin is contacted with the virucidal composition for at most 1 min contact time to reduce the virus titre for at least 4 $\log_{10}$ reduction steps. The virucidal composition for use in the method provides that even after a short contact time the virus is sufficiently inactivated. Particularly, the virucidal composition in the method can be used for virucidal hand disinfection.

The composition can be applied in liquid form, in the form of a gel, as a foam, or as an emulsion. It is preferred that the composition is applied in liquid form. The composition comes into contact with the skin by applying it with the hands followed by rubbing and distributing the composition evenly over the skin. The same application method can be used if the composition is in the form of a gel, in the form of a foam, or in the form of an emulsion. Further, the composition can come into contact with the skin by spraying it onto the skin. The virucidal composition can be applied by using a dispenser or solid support soaked with the virucidal composition. The support can be a woven or non- woven fabric, a textile, a paper towel, cotton wool, an absorbent polymer sheet or a sponge. The composition may be used at any temperature, preferably at ambient temperature.

For a more complete understanding of the invention, the following examples are given to illustrate some embodiments. These examples and experiments are to be understood as illustrative and not limiting.

Example 1

Antiviral efficacy

The following examples were carried out to illustrate the antiviral efficacy of the composition according to the invention.

Table 1

| Compositions of the invention | | |
|---|---|---|
| Components | Example 3 | Example 4 |
| ethanol [wt.-%] | 89 | 85 |
| tocopheryl acetate [wt.-%] | 0.05 | 0.05 |
| myristyl alcohol [wt.-%] | 1 | - |
| panthenol [wt.-%] | 0.146 | 0.146 |
| glycerol [wt.-%] | 0.5 (86%) | 2 (99%) |
| isopropyl palmitate [wt.-%] | - | 0.4 |
| aloe vera [wt.-%] | - | 0.5 |
| ester of benzoic acid and C12-15 alcohols [wt.-%] | - | 0.25 |
| polydimethylsiloxane [wt.-%] | - | 0.05 |
| water, deionized [wt.-%] | Add. 100 | Add. 100 |

Table 2

| Compositions for comparison | | | | |
|---|---|---|---|---|
| Components | Example 1 | Example 2 | Example 5 | Example 6 |
| ethanol [wt.-%] | 89 | 85 | 89 | 89 |
| tocopheryl acetate [wt.-%] | - | - | - | - |
| myristyl alcohol [wt.-%] | - | - | 1 | - |
| panthenol [wt.-%] | - | - | - | - |
| glycerol [wt.-%] | - | - | 0.5 (86%) | 1 (86%) |
| isopropyl palmitate [wt.-%] | - | - | - | - |
| aloe vera [wt.-%] | - | - | - | - |
| ester of benzoic acid and C12-15 alcohols [wt.-%] | - | - | - | - |
| polydimethylsiloxane [wt.-%] | - | - | - | - |
| water, deionized [wt.-%] | Add. 100 | Add. 100 | Add. 100 | Add. 100 |

In table 1 examples 3 and 4 are examples according to the invention with an ethanol concentration of 89 % w/w and 85 % w/w, respectively, and with further components. Comparative examples 1 and 2 are examples only containing 89 % w/w or 85 % w/w ethanol without any further components, while comparative examples 5 and 6 contain 89 % w/w ethanol, myristyl alcohol and glycerol, or ethanol and glycerol.

The compositions of the invention as shown above in Table 1 and the compositions for comparison as shown above in Table 2 were prepared by adding the components to water and mixing.

The antiviral efficacy of the examples against the non-enveloped Polio virus was investigated in a quantitative suspension test. The investigations were performed according to the procedure described in European norm DIN EN 14476 (February 2007).

The virus used was Polio virus Type 1 strain LSc-2ab (Chiron Behring GmbH & Co, Marburg, Germany). Stock virus suspensions were kept frozen at -80°C. The stock viruses were multiplied in the appropriate cell line BGM, kidney cells from African green monkeys (Mikrolab, Bremen, Germany) to produce high titres of infectious viruses. The suspensions with cell detritus were frozen and thawed two times. The cell detritus was then separated by low speed centrifugation. Virus suspensions were stored at -80°C.

A defined suspension of the virus was incubated with the undiluted disinfectant resulting in a dilution of disinfectant in the test mixture and inoculated on cultures of the BGM cell line. After 7 days the infected cells were checked for cytotoxic and cytopathogenic effects. If no cytopathogenic effects were visible, the virus has successfully been inactivated by the disinfectant.

After completion of validation steps, the antiviral activity of the undiluted disinfectant against Polio virus was tested at a time interval of 15, 30, 60, and 120 seconds using phosphate buffered saline (PBS) as interfering substance. At first, one part of interfering substance was mixed with 8 parts of the disinfectant. To this mixture, 1 part of the virus suspension was added, mixed and incubated by vortexing the sample for 15, 30, 60, and 120 seconds. After the incubation time, an aliquot was taken and the virucidal activity was immediately suppressed by serial dilution steps with ice-cold medium. The dilutions were were stored in an ice bath until further processing. At the end of collecting and diluting of the sample, all dilutions were transferred, at 100 μl for each well, into cell culture suspensions and incubated at 37°C ± 1°C. After 7 days the cells were checked for cytopathogenic effects (CPE) and 50% infectious endpoint $TCID_{50}$/ml was determined. The detectable titre reduction should be at least 4 $\log_{10}$. The viral infectivity titre was calculated according to the Spaermann-Karber method:

$$\text{Log}_{10}\ TCID_{50} = - (X_0 - 0.5 + \sum r/n)$$

wherein: $X_0$ = $\log_{10}$ of the lowest dilution step with 100% positive reaction

r = number of all positive reactants of the lowest dilution step with 100% positive reaction and all higher (positive) dilution steps
n = number of all reactants per dilution step.

Furthermore, the invectivity of the test virus suspension was determined under test conditions but without exposure to the disinfectant at a contact time of 120 seconds. Instead of disinfectant water was used.

Based upon the provisions of the European norm DIN EN 14476 (February 2007), virucidal efficacy for a disinfectant is shown by demonstrating a virus titre reduction of at least 4 $\log_{10}$ within the advised exposure time. The following table 3 shows the results of the examples 1 to 6 for the contact times of 15, 30, 60, and 120 seconds.

Table 3

| Inactivation of Polio virus [$\log_{10}$]: | | | | | | |
|---|---|---|---|---|---|---|
| Time | Example 1 | Example 2 | Example 3 | Example 4 | Example 5 | Example 6 |
| 15 sec | < 0.72 | < 0.5 | < 0.72 | < 0.83 | < 0.83 | < 0.83 |
| 30 sec | 2.14 | 0.97 | 3.29 | 1.85 | 1.86 | 2.0 |
| 60 sec | 3.86 | 3.23 | > 4.0 | 3.71 | 3.25 | 3.75 |
| 120 sec | 5.14 | 3.85 | > 4.29 | 5.14 | 4.83 | > 5.0 |

It can be seen in table 3 that for the composition according to example 3 the required titre reduction of at least 4 $\log_{10}$ could be demonstrated already after 60 seconds contact time. Further, the composition according to example 4 showed a titre reduction of 5.14 after 120 seconds of contact time, whereas the respective comparative example 2 also containing 85 % w/w ethanol did not show sufficient reduction in virus titre. This shows that this composition also has adequate virucidal efficacy against Polio virus.

In summary, the above tests show that the compositions according to the invention provide inactivation activity against poliovirus although comprising a low amount of ethanol. The example 3 provides a very fast acting disinfectant.

**Claims**

**1.** A virucidal composition comprising:

from 70 wt.-% to 95 wt.-% of a $C_2$ to $C_3$ monoalcohol,
from 0.01 wt.-% to 0.5 wt.-% of tocopheryl or tocopheryl acetate,
from 0.01 wt.-% to 1.0 wt.-% of panthenol;
from 0.1 wt.-% to 5.0 wt.-% of glycerol; and
from 0.01 wt.-% to 5.0 wt.-% of myristyl alcohol.

**2.** The virucidal composition of claim 1, wherein the virucidal composition further comprises,

from 0.01 wt.-% to 1.0 wt.-% of isopropyl palmitate;
from 0.01 wt.-% to 1.0 wt.-% of aloe vera;
from 0.01 wt.-% to 1.0 wt.-% of $C_{12-15}$ alkyl benzoate; and
from 0.01 wt.-% to 0.5 wt.-% of polydimethylsiloxane.

**3.** The virucidal composition of claim 1, wherein the virucidal composition further comprises from 0.001 wt.-% to 0.5 wt.-%, preferably from 0.001 wt.-% to 0.1 wt.-%, or more preferably from 0.002 wt.-% to 0.01 wt.-% of lactic acid.

**4.** The virucidal composition of claim 1, wherein the virucidal composition further comprises an additive selected from the group consisting of a stabilizer, fragrance, colorant, emulsifier, thickener, neutralizer, denaturant, wetting agent, and mixture thereof.

**5.** The virucidal composition of claim 1, wherein the virucidal composition is in a form of liquid, gel, foam, or emulsion.

**6.** A virucidal composition according to claims 1 to 5 for use in the virucidal disinfection of a virus-contaminated surface of animal or mammal skin, wherein
the virus-contaminated surface of animal or mammal skin is contacted with the virucidal composition for a period of at most 2 min contact time, wherein the virucidal composition reduces virus titre of a virus for at least 4 log10 reduction steps measured according to EN 14476.

7. The virucidal composition for use of claim 6, wherein the contacting further comprises rubbing and/or distributing the composition evenly over the surface of mammal skin.

8. The virucidal composition for use of claim 6, wherein the contacting is by spraying the composition onto the surface of mammal skin, or using a dispenser or solid support soaked with the composition to apply the composition onto the surface of mammal skin.

9. The virucidal composition for use of claim 6, wherein the solid support is a woven or nonwoven fabric, a textile, a paper towel, cotton wool, an absorbent polymer sheet, or a sponge.

10. The virucidal composition for use of claim 6, wherein the surface is skin of human hands.

11. The virucidal composition for use of claim 6, wherein the contact time is less than 1 minute and the virucidal composition reduces viral titre by at least $4\log_{10}$ measured according to EN 14476.

12. The virucidal composition for use of claim 6, wherein the virus is a poliovirus (type 1 strain LSc-2ab).

13. A non-therapeutic method of virucidal disinfection of a virus-contaminated surface, comprising the the steps of:

    providing a virucidal composition according to claims 1 to 5, and
    contacting the virus-contaminated surface with the virucidal composition for a period of at most 2 min contact time, wherein the virucidal composition reduces virus titre of a virus for at least 4 log10 reduction steps measured according to EN 14476.

14. The method of claim 13, wherein the contact time is less than 1 minute and the virucidal composition reduces viral titre by at least $4\log_{10}$ measured according to EN 14476.

15. The method of claim 13, wherein the virus is a poliovirus (type 1 strain LSc-2ab).


**Patentansprüche**

1. Viruzide Zusammensetzung, umfassend:

    zu 70 Gew.-% bis 95 Gew.-% eines $C_2$- bis $C_3$-Monoalkohols,
    zu 0,01 Gew.-% bis 0,5 Gew.-% Tocopheryl oder Tocopherylacetat,
    zu 0,01 Gew.-% bis 1,0 Gew.-% Panthenol;
    zu 0,1 Gew.-% bis 5,0 Gew.-% Glycerin; und
    zu 0,01 Gew.-% bis 5,0 Gew.-% Myristylalkohol.

2. Viruzide Zusammensetzung nach Anspruch 1, wobei die viruzide Zusammensetzung ferner umfasst

    zu 0,01 Gew.-% bis 1,0 Gew.-% Isopropylpalmitat;
    zu 0,01 Gew.-% bis 1,0 Gew.-% Aloe Vera;
    zu 0,01 Gew.-% bis 1,0 Gew.-% $C_{12-15}$-Alkylbenzoat; und
    zu 0,01 Gew.-% bis 0,5 Gew.-% Polydimethylsiloxan.

3. Viruzide Zusammensetzung nach Anspruch 1, wobei die viruzide Zusammensetzung ferner zu 0,001 Gew.-% bis 0,5 Gew.-%, vorzugsweise zu 0,001 Gew.-% bis 0,1 Gew.-% oder mehr bevorzugt zu 0,002 Gew.-% bis 0,01 Gew.-% Milchsäure umfasst.

4. Viruzide Zusammensetzung nach Anspruch 1, wobei die viruzide Zusammensetzung ferner einen Zusatzstoff umfasst, der aus der Gruppe ausgewählt ist, bestehend aus einem Stabilisator, einem Duftstoff, einem Farbstoff, einem Emulgator, einem Verdickungsmittel, einem Neutralisator, einem Denaturierungsmittel, einem Benetzungsmittel und einer Mischung davon.

5. Viruzide Zusammensetzung nach Anspruch 1, wobei die viruzide Zusammensetzung in einer Form von Flüssigkeit, Gel, Schaum oder Emulsion vorliegt.

**6.** Viruzide Zusammensetzung nach den Ansprüchen 1 bis 5 zur Verwendung bei der viruziden Desinfektion einer viruskontaminierten Oberfläche von Tier- oder Säugetierhaut, wobei
die viruskontaminierte Oberfläche der Haut von Tieren oder Säugetieren für einen Zeitraum von höchstens 2 Minuten Berührungszeit mit der viruziden Zusammensetzung in Berührung gebracht wird, wobei die viruzide Zusammensetzung einen Virustiter eines Virus für mindestens 4 log10 Reduktionsschritte reduziert, gemessen gemäß EN 14476.

**7.** Viruzide Zusammensetzung zur Verwendung nach Anspruch 6, wobei das Inberührungbringen ferner ein Reiben und/oder gleichmäßiges Verteilen der Zusammensetzung über die Oberfläche von Säugetierhaut umfasst.

**8.** Viruzide Zusammensetzung zur Verwendung nach Anspruch 6, wobei das Inberührungbringen durch ein Aufsprühen der Zusammensetzung auf die Oberfläche von Säugetierhaut oder durch ein Verwenden eines mit der Zusammensetzung getränkten Spenders oder festen Trägers erfolgt, um die Zusammensetzung auf die Oberfläche der Säugetierhaut aufzutragen.

**9.** Viruzide Zusammensetzung zur Verwendung nach Anspruch 6, wobei der feste Träger ein Gewebe oder Vliesstoff, ein Textilmaterial, ein Papiertuch, Watte, eine absorbierende Polymerbahn oder ein Schwamm ist.

**10.** Viruzide Zusammensetzung zur Verwendung nach Anspruch 6, wobei die Oberfläche Haut von menschlichen Händen ist.

**11.** Viruzide Zusammensetzung zur Verwendung nach Anspruch 6, wobei die Berührungszeit weniger als 1 Minute beträgt und die viruzide Zusammensetzung einen Virustiter um mindestens $4\log_{10}$ reduziert, gemessen gemäß EN 14476.

**12.** Viruzide Zusammensetzung zur Verwendung nach Anspruch 6, wobei das Virus ein Poliovirus (Typ 1-Stamm LSc-2ab) ist.

**13.** Nichttherapeutisches Verfahren für eine viruzide Desinfektion einer viruskontaminierten Oberfläche, umfassend die Schritte:

Bereitstellen einer viruziden Zusammensetzung nach den Ansprüchen 1 bis 5, und
Inberührungbringen der viruskontaminierten Oberfläche mit der viruziden Zusammensetzung für einen Zeitraum von höchstens 2 Minuten Berührungszeit, wobei die viruzide Zusammensetzung einen Virustiter eines Virus für mindestens 4log10 Reduktionsschritte reduziert, gemessen gemäß EN 14476.

**14.** Verfahren nach Anspruch 13, wobei die Berührungszeit weniger als 1 Minute beträgt und die viruzide Zusammensetzung einen Virustiter um wenigstens $4\log_{10}$ reduziert, gemessen gemäß EN 14476.

**15.** Verfahren nach Anspruch 13, wobei das Virus ein Poliovirus (Typ 1-Stamm LSc-2ab) ist.

**Revendications**

**1.** Composition virucide comprenant :

de 70 % en poids à 95 % en poids d'un monoalcool en $C_2$ à $C_3$,
de 0,01 % en poids à 0,5 % en poids de tocophéryle ou d'acétate de tocophéryle,
de 0,01 % en poids à 1,0 % en poids de panthénol ;
de 0,1 % en poids à 5,0 % en poids de glycérol ; et
de 0,01 % en poids à 5,0 % en poids d'alcool myristylique.

**2.** Composition virucide selon la revendication 1, la composition virucide comprenant en outre,

de 0,01 % en poids à 1,0 % en poids de palmitate d'isopropyle ;
de 0,01 % en poids à 1,0 % en poids d'aloe vera ;
de 0,01 % en poids à 1,0 % en poids de benzoate d'alkyle en $C_{12}$ à $C_{15}$ ; et
de 0,01 % en poids à 0,5 % en poids de polydiméthylsiloxane.

**3.** Composition virucide selon la revendication 1, la composition virucide comprenant en outre de 0,001 % en poids à 0,5 % en poids, de préférence de 0,001 % en poids à 0,1 % en poids, ou plus préférablement de 0,002 % en poids à 0,01 % en poids d'acide lactique.

**4.** Composition virucide selon la revendication 1, la composition virucide comprenant en outre un additif choisi dans le groupe constitué d'un stabilisant, d'un parfum, d'un colorant, d'un émulsifiant, d'un épaississant, d'un neutralisant, d'un dénaturant, d'un agent mouillant et de leur mélange.

**5.** Composition virucide selon la revendication 1, la composition virucide étant sous une forme de liquide, de gel, de mousse ou d'émulsion.

**6.** Composition virucide selon les revendications 1 à 5 destinée à être utilisée dans la désinfection virucide d'une surface contaminée par un virus d'une peau d'animal ou de mammifère,
la surface contaminée par un virus de la peau d'un animal ou d'un mammifère étant mise en contact avec la composition virucide pendant une période égale ou inférieure à 2 min de temps de contact, la composition virucide réduisant le titre viral d'un virus pendant au moins 4 étapes de réduction log10 mesurées selon l'EN 14476.

**7.** Composition virucide destinée à être utilisée selon la revendication 6, la mise en contact comprenant en outre le frottement et/ou la distribution de la composition uniformément sur la surface de la peau de mammifère.

**8.** Composition virucide destinée à être utilisée selon la revendication 6, la mise en contact se faisant par pulvérisation de la composition sur la surface de peau de mammifère, ou à l'aide d'un distributeur ou un support solide imbibé de la composition pour appliquer la composition sur la surface de la peau de mammifère.

**9.** Composition virucide destinée à être utilisée selon la revendication 6, le support solide étant un tissu tissé ou non tissé, un textile, une serviette en papier, du coton, une feuille de polymère absorbant ou une éponge.

**10.** Composition virucide destinée à être utilisée selon la revendication 6, la surface étant de la peau de mains humaines.

**11.** Composition virucide destinée à être utilisée selon la revendication 6, le temps de contact étant inférieur à 1 minute et la composition virucide réduisant le titre viral d'au moins 4 $\log_{10}$ mesuré selon EN 14476.

**12.** Composition virucide à utiliser selon la revendication 6, le virus étant un poliovirus (souche de type 1 LSc-2ab).

**13.** Procédé non thérapeutique de désinfection virucide d'une surface contaminée par un virus, comprenant les étapes suivantes :

la fourniture d'une composition virucide selon les revendications 1 à 5, et
la mise en contact de la surface contaminée par le virus avec la composition virucide pendant une période égale ou inférieure à 2 min de temps de contact, la composition virucide réduisant le titre viral d'un virus pendant au moins 4 étapes de réduction log10 mesurées selon l'EN 14476.

**14.** Procédé selon la revendication 13, le temps de contact étant inférieur à 1 minute et la composition virucide réduisant le titre viral d'au moins 4 $\log_{10}$ mesuré selon l'EN 14476.

**15.** Procédé selon la revendication 13, le virus étant un poliovirus (souche de type 1 LSc-2ab).

**REFERENCES CITED IN THE DESCRIPTION**

**Patent documents cited in the description**

- WO 2011072728 A
- EP 0176720 A1
- US 5114957 A
- WO 9961045 A
- US 20080293825 A1
- US 20090226498 A1
- US 20060074029 A1